(19) 

Europäisches
Patentamt

European
Patent Office

Office européen
des brevets

(11) **EP 4 610 353 A1**

(12) **EUROPEAN PATENT APPLICATION**
published in accordance with Art. 153(4) EPC

(43) Date of publication:
**03.09.2025 Bulletin 2025/36**

(21) Application number: **23882780.2**

(22) Date of filing: **27.10.2023**

(51) International Patent Classification (IPC):
*C12N 9/78* (2006.01)   *A23J 3/14* (2006.01)
*A23L 2/00* (2006.01)   *A23L 2/40* (2006.01)
*A23L 2/66* (2006.01)   *A23L 5/00* (2016.01)
*C07K 14/415* (2006.01)   *C12N 9/20* (2006.01)
*C12N 9/50* (2006.01)   *C12P 1/00* (2006.01)

(52) Cooperative Patent Classification (CPC):
**A23J 3/14; A23L 2/00; A23L 2/40; A23L 2/66;
A23L 5/00; C07K 14/415; C12N 9/20; C12N 9/50;
C12N 9/78; C12P 1/00**

(86) International application number:
**PCT/JP2023/038988**

(87) International publication number:
**WO 2024/090579 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.10.2022 JP 2022173787**

(71) Applicants:
• **Amano Enzyme Inc.**
**Nagoya-shi**
**Aichi 460-8630 (JP)**

• **Amano Enzyme U.S.A. Co., Ltd.**
**Elgin, Illinois 60124 (US)**

(72) Inventors:
• **OKUDA, Keita**
**Kakamigahara-shi, Gifu 509-0109 (JP)**
• **BROCHES, Nickolas**
**Elgin, Illinois 60124 (US)**

(74) Representative: **Müller-Boré & Partner**
**Patentanwälte PartG mbB**
**Friedenheimer Brücke 21**
**80639 München (DE)**

(54) **EMULSIFIABILITY IMPROVING AGENT AND FOAMABILITY IMPROVING AGENT FOR LIQUID
COMPOSITION CONTAINING VEGETABLE PROTEIN AND OIL/FAT**

(57) The purpose of the present invention is to provide a processing technique capable of improving the
emulsifiability and/or the foamability of a liquid composition containing a plant protein. It is possible to improve the
emulsifiability and/or the foamability of a liquid composition containing a plant protein and an oil or fat by treating
the liquid composition with a protein deamidase and a
lipase.

EP 4 610 353 A1

**Description**

Technical Field

The present invention relates to a processing technique capable of improving the emulsifiability and/or the foamability of a liquid composition containing a plant protein. Specifically, the present invention relates to an emulsifiability improver or a foamability improver for a liquid composition containing a plant protein and an oil or fat, a method for improving emulsifiability or foamability of a liquid composition containing a plant protein and an oil or fat, a method for producing an emulsion of a liquid composition containing a plant protein and an oil or fat, and an emulsion of a liquid composition containing a plant protein and an oil or fat.

Background Art

In recent years, the milk substitute market and the meat substitute market have been expanding in the food market on the basis of sustainability, health consciousness, and the like. Among them, plant drinks, which are substitutes for animal milks, are one of the important categories in the milk substitute market, and continue to receive attention.

Since the plant drinks have component compositions fundamentally different from those of milks, processing for bringing the characteristics of plant drinks close to those of animal milks is practically essential. Therefore, various processing techniques for plant foods and drinks have been studied.

Examples of the characteristics of the animal milks imitated by the plant drinks include emulsification characteristics. Generally, a drink containing a plant protein is blended with an emulsifier in terms of favorably maintaining the emulsified state, and as such an emulsifier, glycerin fatty acid esters, sorbitan fatty acid esters, propylene glycol fatty acid esters, sucrose fatty acid esters, polyglycerin fatty acid esters, gum arabic, lecithin, and the like are used as described in PTL 1. Meanwhile, a processing technique for performing an emulsification treatment by a method other than an emulsifier has also been studied. For example, PTL 2 describes a method using a plant protein material satisfying prescribed requirements as a technique capable of producing a plant-based liquid nutritional composition having satisfactory emulsion stability without blending milk proteins.

Another example of the properties of animal milks that plant drinks imitate is foamability. For example, PTL 3 describes that good foamability is imparted to soy milk by heat-treating soy milk with reduced fat.

Citation List

Patent Literature

PTL 1: Japanese Patent Laid-open Publication No. 2021-176284
PTL 2: Japanese Patent Laid-open Publication No. 2021-52655
PTL 3: WO 2014/092157 A

Summary of Invention

Technical Problem

Since plant drinks are attracting attention from users who are particularly conscious of health, those blended with no emulsifier as an additive tend to be desired. Currently, however, to impart superior emulsifiability to a plant drink, it is necessary to rely on an emulsifier. In addition, as described in PTL 2, a method using a plant protein material that satisfies a prescribed requirement has also been proposed, but as a requirement of the material, not only the protein content but also the NSI, the molecular weight distribution, and the gelation characteristics all need to satisfy the prescribed ranges. In view of the fact that a material with such strict conditions is not easily available, a method capable of improving emulsifiability using a more easily available material is desired.

Foamability of plant drinks has not been sufficiently studied yet. Although the method described in PTL 3 has been proposed, the method can be applied only to soy milk. In general, it is highly difficult to foam plant drinks. However, in view of the expansion of the future milk substitute market, a method for improving foamability, which method can be applied to other types of plant drinks, is desired.

Therefore, an object of the present invention is to provide a processing technique capable of improving the emulsifiability and/or the foamability of a liquid composition containing a plant protein.

Solution to Problem

The present inventors have found that emulsifiability and/or foamability can be improved by blending an oil or fat into a liquid composition containing a plant protein and treating the resulting composition with a protein deamidase and a lipase. Furthermore, it has also been found that when the treatment is performed using a protease in addition to the protein deamidase and the lipase, emulsifiability and/or foamability can be further improved. The present invention has been accomplished by further studies on the basis of these findings.

In summary, the present invention provides aspects of invention as itemized below.

Item 1. An emulsifiability improver for a liquid composition containing a plant protein and an oil or fat, including a protein deamidase and a lipase.

Item 2. The emulsifiability improver according to item 1, in which an amount of the lipase relative to 1 U of the protein deamidase is 1 U or more.

Item 3. The emulsifiability improver according to item 1 or 2, further including a protease.

Item 4. A foamability improver for a liquid composition containing a plant protein and an oil or fat, including a protein deamidase and a lipase.

Item 5. The foamability improver according to item 4, further including a protease.

Item 6. The foamability improver according to item 5, in which an amount of the protease relative to 1 U of the protein deamidase is 1 U or more.

Item 7. A method for improving emulsifiability of a liquid composition containing a plant protein and an oil or fat, the method including an enzyme treatment step of allowing a protein deamidase and a lipase to act on the liquid composition.

Item 8. The method according to item 7, in which the protein deamidase is used in an amount of 0.05 U or more per 1 g of the plant protein.

Item 9. The method according to item 7 or 8, in which the lipase is used in an amount of 1 U or more per 1 g of the oil or fat.

Item 10. A method for improving foamability of a liquid composition containing a plant protein and an oil or fat, the method including an enzyme treatment step of allowing a protein deamidase and a lipase to act on the liquid composition.

Item 11. A method for producing an emulsion of a liquid composition containing a plant protein and an oil or fat, the method including an enzyme treatment step of treating the liquid composition containing the plant protein and the oil or fat with a protein deamidase and a lipase.

Item 12. The production method according to item 11, in which a protease is further used in the enzyme treatment step.

Item 13. An emulsion of a liquid composition containing a plant protein and an oil or fat, the emulsion being one obtained by the production method according to item 11 or 12 and being a food or drink.

Item 14. Use of an enzyme preparation containing a protein deamidase and a lipase for improving emulsifiability and/or foamability of a liquid composition containing a plant protein and an oil or fat.

Item 15. Application of an enzyme preparation containing a protein deamidase and a lipase as an emulsifiability improver and/or a foamability improver for a liquid composition containing a plant protein and an oil or fat.

Advantageous Effects of Invention

According to the present invention, there is provided a processing technique capable of improving the emulsifiability and/or the foamability of a liquid composition containing a plant protein.

Description of Embodiments

## 1. Emulsifiability improver and foamability improver

Both the emulsifiability improver and the foamability improver for a liquid composition containing a plant protein and an oil or fat of the present invention are characterized by containing a protein deamidase and a lipase. Hereinafter, the emulsifiability improver and the foamability improver of the present invention will be described in detail.

## 1-1. Prescribed enzyme

The emulsifiability improver and the foamability improver of the present invention each contain a protein deamidase and a lipase as active ingredients. From the viewpoint of further improving the effect of improving emulsifiability and the effect of improving foamability, it is preferable that a protease is further contained.

1-1-1. Protein deamidase

The protein deamidase to be used in the present invention is an enzyme that exhibits an action of decomposing an amide group-containing side chain of a protein with being accompanied by neither cleavage of a peptide linkage nor crosslinking of the protein, and the type, origin, or the like of the enzyme are not particularly limited.

Examples of the protein deamidase include an enzyme that deamidates a glutamine residue in a protein and converts it into glutamic acid (for example, protein glutaminase) and an enzyme that deamidates an asparagine residue in a protein and converts it into aspartic acid (for example, protein asparaginase).

More specific examples of the protein deamidase include protein deamidases derived from the genus Chryseobacterium, Flavobacterium, Empedobacter, Sphingobacterium, Aureobacterium, Myroides, Luteimicrobium, Agromyces, Microbacterium, or Leifsonia. These protein deamidases are known, and for example, JP 2000-50887 A, JP 2001-218590 A, WO 2006/075772 A1, and WO 2015/133590 can be referred to. These protein deamidases may be used singly or two or more of them may be used in combination.

Among these protein deamidases, protein glutaminases are preferable from the viewpoint of further improving the effect of improving emulsifiability and/or the effect of improving foamability, protein glutaminases derived from the genus Chryseobacterium are more preferable, and a protein glutaminase derived from Chryseobacterium proteolyticum is still more preferable.

The protein deamidase can be prepared from a culture solution of a microorganism from which the protein deamidase is to be derived. Examples of a specific preparation method include a method of collecting a protein deamidase from a culture solution or bacterial cells of the microorganism. For example, in the case of using a microorganism that secretes a protein deamidase, the enzyme can be separated and/or purified after collecting bacterial cells from the culture solution in advance by filtration, centrifugation or the like as necessary. In the case of using a microorganism that does not secret a protein deamidase, after bacterial cells are collected from the culture solution in advance, as necessary, the bacterial cells are disrupted by pressurization treatment, ultrasonic treatment, or the like to expose the enzyme, and then the enzyme can be separated and/or purified. As an enzyme separation and/or purification method, a known protein separation and/or purification method can be used without particular limitation, and examples thereof include a centrifugal separation method, a UF concentration method, a salting-out method, and various chromatography methods using an ion exchange resin. The separated and/or purified enzyme can be pulverized by a drying method such as freeze-drying or reduced-pressure drying, and can also be pulverized using an appropriate excipient and/or drying aid in the drying method. The separated and/or purified enzyme can also be liquefied by adding an appropriate additive and subjecting it to filtration sterilization.

The amount of the protein deamidase contained in the emulsifiability improver and the foamability improver of the present invention is not particularly limited as long as the effect of the protein deamidase can be effectively obtained when the emulsifiability improver and the foamability improver of the present invention are used, and is, for example, 0.1 to 5,000 U/g.

For the activity of the protein deamidase, the amount of enzyme liberating 1 $\mu$mol of ammonia per minute using benzyloxycarbonyl-L-glutaminylglycine (Z-Gln-Gly) as a substrate is defined as 1 unit (1 U).

1-1-2. Lipase

The lipase to be used in the present invention is not particularly limited in terms of its type, origin, etc. as long as the lipase is an enzyme that acts to hydrolyze triglycerides.

Examples of the lipase include lipases derived from the genus Rhizopus, Aspergillus, Mucor, Rhizomucor, Thermomyces, Pseudomonas, Geotrichum, Penicillium, or Candida.

Examples of the lipase derived from the genus Rhizopus include lipases derived from Rhizopus delemar, Rhizopus oryzae, Rhizopus arrhizus, Rhizopus niveus, or Rhizopus javanicus. Examples of the lipase derived from the genus Aspergillus include a lipase derived from Aspergillus niger. Examples of the lipase derived from the genus Mucor include lipases derived from Mucor javanicus, or Mucor miehei. Examples of the lipase derived from the genus Rhizomucor include a lipase derived from Rhizomucor miehei. Examples of the lipase derived from the genus Thermomyces include a lipase derived from Thermomyces lanuginosus.

These lipases may be used singly or two or more of them may be used in combination. Among these lipases, lipases derived from the genus Rhizopus are preferable from the viewpoint of further improving the effect of improving emulsifiability and/or the effect of improving foamability, and a lipase derived from Rhizopus oryzae is more preferable.

In addition, from the viewpoint of further improving the effect of improving emulsifiability and/or the effect of improving foamability, the lipase to be used in the present invention is preferably an enzyme having an action of hydrolyzing an ester linkage at one or two positions (specifically, the 1-position, the 2-position, the 3-position, the 1-position and the 2-position, the 1-position and the 3-position, or the 2-position and the 3-position) among the 1 ($\alpha$)-position, the 2($\beta$)-position, and the 3($\gamma$)-position of triglyceride, more preferably an enzyme having an action of hydrolyzing ester linkages at the 1-position and the 3-position of triglyceride, and still more preferably an enzyme having an action of hydrolyzing ester linkages at the 1-position and the 3-position of triglyceride in a position-specific manner.

The amount of the lipase contained in the emulsifiability improver and the foamability improver of the present invention is not particularly limited as long as the effect of the lipase can be effectively obtained when the emulsifiability improver and the foamability improver of the present invention are used, and is, for example, 1 to 1,500,000 U/g.

In the emulsifiability improver and the foamability improver of the present invention, the amount of the lipase per 1 U of the protein deamidase is, for example, 0.1 U or more, 0.3 U or more, 0.5 U or more, or 1 U or more, and from the viewpoint of further improving the effect of improving emulsifiability and/or the effect of improving foamability, the amount of the lipase is preferably 2 U or more, 2.5 U or more, 3 U or more, 4 U or more, 7 U or more, or 10 U or more, and more preferably 12 U or more, 13 U or more, or 14 U or more. The upper limit of the range of the amount of the lipase per 1 U of the protein deamidase is not particularly limited, and is, for example, 1000 U or less or 600 U or less, and from the viewpoint of further improving the effect of improving emulsifiability and/or the effect of improving foamability, is preferably 300 U or less, 200 U or less, 160 U or less, 150 U or less, 140 U or less, or 120 U or less, more preferably 100 U or less, 90 U or less, 80 U or less, or 75 U or less, and still more preferably 70 U or less, 60 U or less, 50 U or less, 40 U or less, 30 U or less, or 20 U or less.

For the activity of the lipase, the amount of the enzyme that affords an increase in the amount of a free fatty acid of 1 $\mu$mol per minute using olive oil as a substrate is defined as 1 unit (1 U).

<u>1-1-3. Protease</u>

The type, origin, etc. of the protease to be used in the present invention are not particularly limited. Preferably, an endopeptidase is used as the protease.

Examples of the protease include a protease derived from a filamentous fungus and a protease derived from a bacterium.

The protease derived from a filamentous fungus is not particularly limited, and examples thereof include proteases derived from the genera Aspergillus, Rhizopus, Mucor, Neurospora, Penicillium, Rhizomucor, and Sclerotinia.

Examples of the protease derived from the genus Aspergillus include proteases derived from Aspergillus oryzae, Aspergillus niger, Aspergillus melleus, Aspergillus japonicus, Aspergillus awamori, Aspergillus kawachii, Aspergillus sojae, Aspergillus tamarii, Aspergillus foetidus, Aspergillus fumigatus, Aspergillus nidulans, Aspergillus aculeatus, Aspergillus candidus, Aspergillus flavus, Aspergillus saitoi, Aspergillus inuii, Aspergillus glaucus, Aspergillus caesiellus, Aspergillus clavatus, Aspergillus deflectus, Aspergillus fischerianus, Aspergillus parasiticus, Aspergillus penicillioides, Aspergillus restrictus, Aspergillus sydowii, Aspergillus terreus, Aspergillus ustus, and Aspergillus versicolor.

Examples of the protease derived from the genus Rhizopus include proteases derived from Rhizopus chinensis, Rhizopus delemar, Rhizopus niveus, and Rhizopus oryzae.

Examples of the protease derived from a bacterium include proteases derived from the genera Bacillus and Geobacillus.

Examples of the protease derived from the genus Bacillus (or Geobacillus) include proteases derived from Bacillus amyloliquefaciens, Bacillus cereus, Bacillus clausii, Bacillus intermedius, Bacillus lentus, Bacillus licheniformis, Bacillus stearothermophilus, Bacillus subtilis, and Bacillus thermoproteolyticus, and these species belonging to the genus Geobacillus.

In the present invention, the protease described above may be used singly or two or more of them may be used in combination. Among the above proteases, proteases derived from filamentous fungi are preferable from the viewpoint of further improving the effect of improving emulsifiability and/or the effect of improving foamability, proteases derived from the genus Aspergillus are more preferable, and a protease derived from Aspergillus oryzae is further preferable.

The amount of the protease contained in the emulsifiability improver and the foamability improver of the present invention is not particularly limited as long as the effect of the protease can be effectively obtained when the emulsifiability improver and the foamability improver of the present invention are used, and is, for example, 1 to 500,000 U/g.

When the emulsifiability improver and the foamability improver of the present invention contain a protease, the amount of the protease per 1 U of the protein deamidase is, for example, 0.1 U or more, 0.3 U or more, 0.5 U or more, or 1 U or more, and from the viewpoint of further improving the effect of improving emulsifiability and/or the effect of improving foamability, the amount of the protease per 1 U of the protein deamidase is preferably 2 U or more, more preferably 3 U or more, and still more preferably 3.5 U or more. The upper limit of the range of the amount of the protease per 1 U of the protein deamidase is not particularly limited, and it is, for example, 150 U or less. From the viewpoint of further improving the effect of improving emulsifiability and/or the effect of improving foamability, the upper limit is preferably 100 U or less, 75 U or less, or 50 U or less, more preferably 25 U or less, or 15 U or less, still more preferably 10 U or less, further preferably 5 U or less, and still further preferably 4 U or less.

For the protease activity, the amount of the enzyme that affords an increase in the amount of a Folin test solution-coloring substance corresponding to 1 $\mu$g of tyrosine per minute using casein as a substrate is 1 unit (1 U).

1-2. Other components

The emulsifiability improver and the foamability improver of the present invention each can be prepared as an enzyme preparation containing the above-described prescribed enzyme, and each may contain components other than the above-described prescribed enzyme to an extent where the effects of the present invention are not affected. Examples of such other components include enzymes other than the above-described prescribed enzymes, and/or bases and/or additives used in ordinary enzyme preparations. Such other components may be appropriately determined according to the use, the preparation form, etc. of the emulsifiability improver and the foamability improver.

1-3. Preparation form

The preparation forms of the emulsifiability improver and the foamability improver of the present invention may be liquid or solid.

1-4. Applications

The emulsifiability improver and the foamability improver of the present invention are used for the purpose of improving the emulsifiability and the foamability of a liquid composition containing a plant protein and an oil or fat, respectively. Specifically, the emulsifiability improver and the foamability improver of the present invention can be used in the enzyme treatment steps in the methods described below.

A method for improving emulsifiability of a liquid composition containing a plant protein and an oil or fat, the method including an enzyme treatment step of allowing a protein deamidase and a lipase to act on the liquid composition;

a method for improving foamability of a liquid composition containing a plant protein and an oil or fat, the method including an enzyme treatment step of allowing a protein deamidase and a lipase to act on the liquid composition; or a method for producing an emulsion of a liquid composition containing a plant protein and an oil or fat, the method including an enzyme treatment step of treating the liquid composition containing the plant protein and the oil or fat with a protein deamidase and a lipase.

Details of the liquid compositions, the enzyme treatment steps, etc. in the aforementioned methods will be described in detail in the following "2. Method for improving emulsifiability of liquid composition containing plant protein and oil or fat, and method for improving foamability of liquid composition containing plant protein and oil or fat".

2. Method for improving emulsifiability of liquid composition containing plant protein and oil or fat, and method for improving foamability of liquid composition containing plant protein and oil or fat

Both the method for improving emulsifiability of a liquid composition containing a plant protein and an oil or fat and the method for improving foamability of a liquid composition containing a plant protein and an oil or fat of the present invention are characterized by including an enzyme treatment step of allowing a protein deamidase and a lipase to act on a liquid composition containing a plant protein and an oil or fat. Hereinafter, these methods of the present invention will be described in detail.

2-1. Liquid composition containing plant protein and oil or fat

The liquid composition to be subjected to an enzyme treatment in each of the methods of the present invention contains a plant protein and an oil or fat.

2-1-1. Plant protein

Examples of the plant protein include, but are not particularly limited to, proteins of legumes such as soybean, pea, lupine bean, broad bean, chickpea, mung bean, and kidney bean; proteins of cereals such as oats, barley, wheat, rye, rice, buckwheat, barnyard millet, foxtail millet, teff, quinoa, and corn; seeds such as canary seed, linseed, almond, cashew nut, hazelnut, pecan nut, macadamia nut, pistachio, walnut, Brazil nut, peanut, coconut, hemp (industrial hemp), pilinut, chestnut, sesame, and pine nut. **In** addition, these proteins may be in any form among the form of a natural product, the form of a protein chemically partially decomposed by an acid, an alkali or the like, the form of a protein chemically modified by various reagents, and the form of a synthetic protein. When the plant protein is in the form of a natural product, the plant protein may be contained in association with the use, as a material of the liquid composition, of a plant material (specifically, the aforementioned legumes, cereals, and seeds) from which the plant protein is derived, or may be contained by using, as a material of the liquid composition, a processed material in which the protein content is increased to an arbitrary level (for example, to an extent that the protein content is 20 to 100% by weight) from the plant material. The form of the processed material is not particularly limited, and examples thereof include a powder form, a slurry form, and a liquid form.

In the present invention, the plant proteins described above may be used singly or two or more of them may be used in combination. From the viewpoint of further improving emulsifiability and/or foamability, legume proteins and seed proteins are preferable, proteins of soybean, pea, lupine bean, broad bean, chickpea, mung bean, kidney bean, canary seed, linseed, almond, cashew nut, hazelnut, pecan nut, macadamia nut, pistachio, walnut, Brazil nut, peanut, coconut, hemp (industrial hemp), pilinut, chestnut, sesame, and pine nut are more preferable, and proteins of pea and almond are still more preferable.

The content of the plant protein in the liquid composition is not particularly limited, and is, for example, 0.1 to 20% by weight, preferably 0.2 to 10% by weight, and more preferably 0.35 to 4% by weight.

The content of the plant material or the processed material contained in the liquid composition (note that when these materials contain water, the content does not include the content of the water) is, for example, 0.1 to 50% by weight, preferably 0.2 to 35% by weight, and more preferably 0.4 to 20% by weight.

2-1-2. Oil or fat

The oil or fat is not particularly limited, but a plant oil or fat is typically used. Examples of the plant oil or fat include oils or fats of legumes such as soybean, pea, lupine bean, broad bean, chickpea, mung bean, and kidney bean; oils or fats of cereals such as oats, barley, wheat, rye, rice, buckwheat, Japanese millet, foxtail millet, teff, quinoa, and corn; and oils or fats of seeds such as rape seed, sunflower seed, camellia seed, safflower seed, cotton seed, oil palm fruit, palm tree fruit, cacao, avocado, olive, canary seed, linseed, shea nut, almond, cashew nut, hazelnut, pecan nut, macadamia nut, pistachio, walnut, Brazil nut, peanut, coconut, hemp (industrial hemp), pili nut, chestnut, sesame, and pine nut. As the oil or fat, an animal oil or fat may be used. Examples of the animal oil or fat include fish oil, lard, beef tallow, and milk fat. In addition, these oils or fats are in the form of a natural product; the form of a hydrogenated oil; the form of a fractionated oil; and the form of a synthetic oil or fat prepared by transesterification or the like. When the oil or fat is a plant oil or fat and is in the form of a natural product, the plant oil or fat may be contained in association with the use, as a material of the liquid composition, of a plant material (specifically, the aforementioned legumes, cereals, and seeds) from which the plant oil or fat is derived, or may be contained by using, as a material of the liquid composition, a processed material in which the oil or fat content is increased to an arbitrary level (for example, to an extent that the oil or fat content is 40 to 100% by weight) from the plant material. The form of the processed material is not particularly limited, and examples thereof include a powder form, a slurry form (namely, the form of a butter), and a liquid form.

In the present invention, the oils or fats described above may be used singly or two or more of them may be used in combination. From the viewpoint of further improving emulsifiability and/or foamability, plant oils or fats are preferable, oils or fats of soybean, pea, rice, corn, rapeseed, sunflower seed, safflower seed, cotton seed, oil palm fruit, palm tree fruit, cacao fruit, almond, cashew nut, and peanut are more preferable, and oils or fats of pea, rapeseed, sunflower seed, and almond are further preferable.

When the oil or fat is a plant oil or fat, the plant from which the plant oil or fat is derived may be the same as or different from the plant from which the plant protein is derived, or may be a combination thereof.

The content of the oil or fat in the liquid composition is not particularly limited, and may be appropriately determined according to the type of the desired emulsified composition and/or the desired foamable composition, and is, for example, 0.1 to 25% by weight, and from the viewpoint of further improving the effect of improving emulsifiability and/or the effect of improving foamability, the content is preferably 1 to 22% by weight, more preferably 3 to 20% by weight, still more preferably 4.8 to 18% by weight, and further preferably 10 to 17% by weight or 13 to 16% by weight.

The ratio between the content of the plant protein and the content of the oil or fat is determined according to each content described above, and the content of the oil or fat per 1 part by weight of the plant protein is preferably 0.1 to 60 parts by weight, more preferably 0.5 to 50 parts by weight, still more preferably 10 to 47 parts by weight, and further preferably 20 to 45 parts by weight or 35 to 40 parts by weight.

When the oil or fat is a plant oil or fat, the content of the plant material or the processed material contained in the liquid composition (note that when these materials contain water, the content does not include the content of the water) is, for example, 0.1 to 40% by weight, preferably 1 to 35% by weight, more preferably 2.5 to 30% by weight, still more preferably 5 to 25% by weight, and further preferably 10 to 20% by weight or 13 to 17% by weight.

2-1-3. Other components

The liquid composition may appropriately contain a seasoning, an emulsifier, a gelling agent, etc. in addition to the above-described components and water. Examples of the seasoning include sugar and common salt, and sugar is preferable. When the liquid composition contains sugar, the content of sugar is, for example, 0.1 to 1.5% by weight, preferably 0.3 to 1.2% by weight, and more preferably 0.5 to 1% by weight. Examples of the emulsifier include those used as an emulsifier in alternative drinks of animal milks, and specific examples thereof include glycerin fatty acid esters, sorbitan fatty acid esters, propylene glycol fatty acid esters, sucrose fatty acid esters, polyglycerin fatty acid esters, gum arabic, and lecithin.

Each of the emulsifiability improver and the foamability improver of the present invention is superior in the effect of improving emulsifiability and the effect of improving foamability of the liquid composition, and thus as a suitable example of the liquid composition, it preferably does not contain an emulsifier (particularly lecithin) and/or a gelling agent, and more preferably contains neither an emulsifier (particularly lecithin) nor a gelling agent.

### 2-1-4. Form

The form of the liquid composition is not particularly limited as long as it is a liquid. For example, the liquid composition may be in the form of an emulsified composition or may be in the form other than an emulsified composition. The liquid composition may be in the form of a foamed composition or may be in the form of a non-foamed composition. Since each of the emulsifiability improver and the foamability improver of the present invention is superior in the effect of improving emulsifiability and the effect of improving foamability of a liquid composition, a preferred example is the form of the liquid composition include the form of the composition that is not an emulsified composition and/or is not a foamed composition, and a more preferred example is the form of a composition that is not an emulsified composition and is not a foamed composition.

### 2-1-5. Preparation method

The method for preparing a liquid composition is not particularly limited as long as the liquid composition is prepared as a composition containing a plant protein and an oil or fat in water. Examples of the preparation method include a method of preparing a liquid composition by mixing water and a processed material resulting from a plant material and containing a plant protein and a plant oil or fat (and other components as necessary); a method of preparing a liquid composition by mixing water, a processed material resulting from a plant material containing at least a plant protein, and a processed material resulting from a plant material containing at least a plant oil or fat (and other components as necessary); a method of crushing and dispersing a plant material containing a plant protein and a plant oil or fat in water, and mixing other components as necessary for preparation. Examples of the method include a method in which a plant material containing at least a plant protein is crushed and dispersed in water, and an oil or fat and, if necessary, other components are mixed and prepared.

### 2-2. Enzyme treatment step

In the enzyme treatment step, a protein deamidase and a lipase are allowed to act on the liquid composition described above. From the viewpoint of further improving the effect of improving emulsifiability and/or the effect of improving foamability, it is preferable to use a protease in combination with the protein deamidase and the lipase.

### 2-2-1. Prescribed enzyme

Details of specific examples of the protein deamidase, the lipase, and the protease are as described above in "1-1-1. Protein deamidase", "1-1-2. Lipase", and "1-1-3. Protease", respectively.

The use ratios of the lipase and the protease to the protein deamidase are as described above in "1-1-2. Lipase" and "1-1-3. Protease", respectively.

The specific amount of the protein deamidase to be used is not particularly limited, but is, for example, 0.05 U or more or 0.1 U or more per 1 g of the plant protein, and from the viewpoint of further improving the effect of improving emulsifiability and/or the effect of improving foamability, it is preferably 0.4 U or more, more preferably 1 U or more, 2 U or more, 3 U or more, 3.5 U or more, or 4 U or more, still more preferably 4.5 U or more, and further preferably 4.8 U or more. The upper limit of the range of the amount of the protein deamidase to be used is not particularly limited, and it is, for example, 50 U or less, 40 U or less, 30 U or less, 20 U or less, or 15 U or less, preferably 10 U or less, and more preferably 7 U or less, or 6 U or less per 1 g of the plant protein.

The specific amount of the lipase to be used is not particularly limited, and is, for example, 1 U or more per 1 g of the oil or fat, and from the viewpoint of further improving the effect of improving emulsifiability and/or the effect of improving foamability, it is preferably 5 U or more, 10 U or more, 14 U or more, 30 U or more, 40 U or more, or 50 U or more, and more preferably 60 U or more, 65 U or more, 70 U or more, or 73 U or more. The upper limit of the range of the amount of the lipase to be used is not particularly limited, and is, for example, 500 U or less, 400 U or less, 350 U or less, or 300 U or less per 1 g of the oil or fat, and from the viewpoint of further improving the effect of improving emulsifiability and/or the effect of improving foamability, it is preferably 200 U or less, or 170 U or less, more preferably 130 U or less, or 120 U or less, and further preferably 90 U or less, 85 U or less, or 80 U or less.

In the present invention, when a protease is further combined, the specific amount of the protease to be used is not particularly limited, and the amount per 1 g of the plant protein is, for example, 1 U or more, and from the viewpoint of further improving the effect of improving emulsifiability and/or the effect of improving foamability, it is preferably 5 U or more, more preferably 10 U or more, and still more preferably 13 U or more. The upper limit of the range of the amount of the protease to be used is not particularly limited, and is, for example, 150 U or less, 100 U or less, or 75 U or less per 1 g of the plant protein, and from the viewpoint of further improving the effect of improving emulsifiability and/or the effect of improving foamability, it is preferably 60 U or less, more preferably 50 U or less, still more preferably 40 U or less, further preferably 30 U or less, and still further preferably 20 U or less, or 17 U or less.

2-2-2. Treatment conditions, etc.

In the enzyme treatment step, a reaction for improving emulsifiability and foamability in the liquid composition is advanced by the treatment.

The order in which the enzymes are allowed to act is not particularly limited, and the enzymes may be allowed to act sequentially in any order, or all the enzymes may be allowed to act simultaneously, and preferably all the enzymes may be allowed to act simultaneously.

The reaction conditions (temperature, pH, time, etc.) in the enzyme treatment are appropriately selected according to the temperature characteristics and the pH characteristics of the enzymes to be used, the scale of the liquid composition, the degree of the intended effect of improving emulsifiability or foamability, etc.

The reaction temperature can be appropriately determined by those skilled in the art according to the temperature characteristics, etc. of the enzymes to be used, and is, for example, 20°C to 60°C, preferably 35°C to 56°C, and more preferably 45°C to 52°C.

The reaction pH (the pH of the liquid composition) can be appropriately determined by those skilled in the art according to the pH characteristics, etc. of the enzymes to be used, and the pH at 25°C is, for example, 5 to 8 or 5 to 7, preferably 5.5 to 7, more preferably 6 to 6.9, and further preferably 6.3 to 6.9.

The treatment time can be appropriately determined by those skilled in the art according to the scale of the liquid composition, the degree of the intended effect of improving emulsifiability or foamability, etc., and is, for example, 10 minutes to 4 hours, and preferably 0.5 hours to 3 hours.

It is preferable to stir the reaction liquid during the enzyme treatment step. The stirring speed can be appropriately determined by those skilled in the art according to the scale of the liquid composition, etc., and is, for example, 100 to 300 rpm, preferably 150 to 250 rpm, and more preferably 180 to 220 rpm.

2-3. Other steps

The method of the present invention may include any other step in addition to the above-described enzyme treatment step. Examples of such other steps include a step of preparing the liquid composition described above as a step to be performed before the enzyme treatment step, and an emulsification step (in the case of a method for improving emulsifiability and in the case of a method for improving foamability) and a foaming step (in the case of a method for improving foamability) as steps to be performed after the enzyme treatment step. Details of the step of preparing the liquid composition are as described above in "2-1-5. Preparation method". The conditions of the emulsification step include the above reaction temperature as a temperature, a stirring condition of, for example, 3000 to 7000 rpm, preferably 4000 to 6000 rpm, more preferably 4500 to 5500 rpm, and a stirring time of, for example, 2 to 20 minutes, preferably 5 to 15 minutes, and more preferably 8 to 13 minutes. As the conditions of the foaming step, conditions generally used for foaming using an animal milk may be appropriately adopted.

3. Method for producing emulsion of liquid composition containing plant protein and oil or fat

The method for producing an emulsion of a liquid composition containing a plant protein and an oil or fat of the present invention is characterized by including an enzyme treatment step of treating a liquid composition containing a plant protein and an oil or fat with a protein deamidase and a lipase.

Details of the liquid composition, the enzyme treatment step, and other steps are as described above in "2. Method for improving emulsifiability of liquid composition containing plant protein and oil or fat, and method for improving foamability of liquid composition containing plant protein and oil or fat".

## 4. Emulsion of liquid composition containing plant protein and oil or fat

The emulsion of a liquid composition containing a plant protein and an oil or fat of the present invention is one being an emulsified composition obtained by the above "3. Method for producing emulsion of liquid composition containing plant protein and oil or fat" and being a food or drink. The emulsion of the present invention has superior emulsifiability and foamability.

The emulsion type of the emulsion of the present invention may be either an oil-in-water type or a water-in-oil type, and an oil-in-water type is preferable.

Examples of a specific form of the food or drink according to the emulsion of the present invention include a plant protein drink (plant milk, etc.), a plant creamer (coffee creamer, etc.), a plant foamed milk, and a plant cream.

Examples

Hereinafter, the present invention will be specifically described with reference to Examples, but the present invention is not to be construed as being limited to the following Examples.

[Materials used]

Details of the materials (enzymes, substrates, and additives) used in the following Test Examples are as follows.

[Table 1]

| Name of material | Product name | Content of component | Manufacturer |
|---|---|---|---|
| Protein deamidase | Protein glutaminase derived from Chryseobacterium proteolyticum | | Amano Enzyme Inc. |
| Lipase | Lipase derived from Rhizopus oryzae (enzyme having an action of site-specifically hydrolyzing ester linkages at positions 1 and 3 of glyceride) | | Amano Enzyme Inc. |
| Protease | Protease derived from Aspergillus oryzae | | Amano Enzyme Inc. |
| Almond butter | Blanched and Roasted AVOLA Almond Paste | Protein content: 22.4% by weight Oil content: 48.8% by weight | Di Bartolo |
| Pea protein material | PURIS PEA870 | Protein content: 80% by weight | PURIS |
| Rapeseed oil | Non-GMO, Canola Oil | Oil content: 100% by weight | NATIVE HARVEST |
| Sunflower oil | Sunflower Oil | Oil content: 100% by weight | Meijer |
| Lecithin | Sunflower Liquid Lecithin | | NOW foods |
| Cane sugar | PURE CANE SUGAR | | IBERIA |

[Method for measuring enzyme activity]

(1) Method for measuring protein deamidase activity

To 1 mL of a 0.2 M phosphate buffer (at pH 6.5) containing 30 mM Z-Gln-Gly, 0.1 mL of a sample solution containing a protein deamidase was added, the mixture was allowed to stand at 37°C for 10 minutes, and then 1 mL of a 0.4 M TCA solution was added to stop the reaction. To 1 mL of a 0.2 M phosphate buffer (at pH 6.5) containing 30 mM Z-Gln-Gly, 1 mL of a 0.4 M TCA reagent was added, 0.1 mL of a sample solution containing a protein deamidase was further added, and the mixture was allowed to stand at 37°C for 10 minutes as a blank.

The solution obtained above was measured using Ammonia Test Wako (FUJIFILM Wako Pure Chemical Corporation) to determine the amount of ammonia generated in the reaction liquid. A calibration curve representing the relationship between the ammonia concentration and the absorbance (630 nm) was prepared using an ammonia standard solution (ammonium chloride), and from the calibration curve, the ammonia concentration in the reaction liquid was determined.

The amount of the enzyme that produces 1 μmol of ammonia per minute was defined as 1 unit (1 U), and the activity of the protein deamidase was calculated from the following formula. In the formula, the reaction liquid amount is 2.1, the enzyme solution amount is 0.1, and Df is a dilution rate of the enzyme solution. 17.03 is a molecular weight of ammonia.

[Equation 1]

Protein deamidase activity (U/mL)

= ammonia concentration in reaction liquid (mg/L) × (1/17.03) × (reaction

liquid amount/enzyme solution amount) × (1/10) × Df

(2) Method for measuring lipase activity

A neutral olive oil emulsion as a substrate was prepared by the following method. In 400 mL of water was dissolved 200 g of anhydrous sodium carbonate, and the resulting solution was added to about 1.5 L of olive oil having been warmed at 45°C for 20 minutes in advance, with gentle stirring such that emulsification did not occur, and the resulting mixture was further stirred at room temperature for about 15 minutes. The resulting mixture was allowed to stand at room temperature for 20 hours or more, and then the oil was centrifuged (5°C, 5,000 min$^{-1}$, 20 min) to prepare a neutral olive oil. A homogenizer vessel was charged with 51.4 mL of the neutral olive oil and 158 mL of a 11% by weight gum arabic/1.25% by weight calcium chloride dihydrate solution, and the mixture was emulsified at 16,000 ± 500 min$^{-1}$ for 30 minutes to prepare a neutral olive oil emulsion.

9 mL of water, 2 mL of a 0.5% by weight sodium taurocholate test solution, and 24 mL of the neutral olive oil emulsion as a substrate were put into a flat bottom test tube and mixed, and the resulting mixture was allowed to stand at 37 ± 0.5°C for 10 to 15 minutes. A pH electrode and a 0.02 mol/L sodium hydroxide solution (for quantitative determination) injection tube were immersed in this solution, and then the solution was continuously stirred. The pH of the solution was adjusted to 7.00 with a 0.02 mol/L sodium hydroxide solution (for quantitative determination), and the burette was set to "0". 5 mL of a sample solution containing a lipase was added to initiate a reaction. A 0.02 mol/L sodium hydroxide solution (for quantitative determination) was continuously added dropwise so as to maintain a pH of 7.00, and after 10 minutes, a 0.02 mol/L sodium hydroxide solution (for quantitative determination) was added to adjust the pH to 9.00. The amount (V10 (mL)) of the 0.02 mol/L sodium hydroxide solution (for quantitative determination) used was recorded. The blank solution was also operated in the same manner to adjust the pH to 9.00, then 5 mL of a sample solution containing a lipase was added, the pH was adjusted to 9.00 again, and the amount (V0 (mL)) of the 0.02 mol/L sodium hydroxide solution (for quantitative determination) used was recorded.

Under these conditions, the amount of an enzyme that leads to an increase in free fatty acid of 1 μmol per minute was defined as 1 unit (1 U), and the amount was calculated from the following equation.

[Equation 2]

Lipase activity (U/g, U/mL) = (V10 - V0) × f × n × 0.4

V0: Titration value (mL) of blank
V10: Titration value (mL) of reaction solution
f: Factor of 0.02 mol/L sodium hydroxide solution (for quantitative determination)
n: Dilution factor per 1 g or 1 mL of sample

(3) Method for measuring protease activity

5 mL of a 0.6% (w/v) casein solution (0.7% (w/v) lactic acid, at pH 3.0) was warmed at 37°C for 10 minutes, 1 mL of a sample solution containing a protease was then added, and the mixture was immediately shaken. This solution was allowed to stand at 37°C for 10 minutes, 5 mL of a 0.44 mol/L trichloroacetic acid test solution was then added, the mixture was shaken, and again allowed to stand at 37°C for 30 minutes, and the mixture was filtered. The first filtrate (3 mL) was removed, and the next filtrate (2 mL) was weighed. Then, 5 mL of a 0.55 mol/L sodium carbonate test solution and 1 mL of a Folin test solution (1 → 3) were added, and the mixture was shaken well and then allowed to stand at 37°C for 30 minutes. For this liquid (enzyme reaction liquid), an absorbance AT at a wavelength of 660 nm was measured using water as a control. Separately, an absorbance AB was measured for a liquid (blank) that was operated in the same manner as the above-described enzyme reaction liquid except that 1 mL of a sample solution containing a protease was weighed, 5 mL of a 0.44 mol/L trichloroacetic acid test solution was added, and the mixture was shaken, then 5 mL of a 0.6% (w/v) casein solution (0.7% (w/v) lactic acid, at pH 3.0) was added, and the mixture was immediately shaken and then allowed to stand at 37°C for 30 minutes. The amount of an enzyme that afforded an increase in the amount of a Folin test solution-coloring substance corresponding to 1 μg of tyrosine per minute was defined as 1 unit (1 U).

1 mL, 2 mL, 3 mL, and 4 mL of a 1 mg/mL tyrosine standard stock solution (0.2 mol/L hydrochloric acid) were each weighed, and a 0.2 mol/L hydrochloric acid test solution was added thereto to make 100 mL. To 2 mL of each solution, 5 mL of a 0.55 mol/L sodium carbonate test solution and 1 mL of a Folin test solution (1 → 3) were added, the mixture was immediately shaken, and the mixture was then allowed to stand at 37°C for 30 minutes. For these liquids, 2 mL of a 0.2 mol/L hydrochloric acid test solution was weighed, and absorbances A1, A2, A3 and A4 at a wavelength of 660 nm were measured using a liquid obtained by operating in the same manner as described above as a control. A calibration curve was created by plotting the absorbances A1, A2, A3, and A4 on the vertical axis and plotting the amount of tyrosine (μg) in 2 mL of each liquid on the horizontal axis, and thus the amount of tyrosine (μg) with respect to the absorbance difference 1 was determined.

[Equation 3]

$$\text{Protease activity (U/g, U/mL)} = (AT - AB) \times F \times 11/2 \times 1/10 \times 1/M$$

AT: Absorbance of enzyme reaction solution
AB: Absorbance of blank
F: Amount (μg) of tyrosine when absorbance difference determined from tyrosine calibration curve is 1
11/2: Conversion factor to total amount of liquid after stop of reaction
1/10: Conversion factor to per minute of reaction time
M: Amount (g or mL) of sample in 1 mL of sample solution

[Test Example 1]

Rapeseed oil was added to a protein solution prepared by suspending a pea protein material in a 0.1 M sodium phosphate buffer solution (at pH 6.8), and the mixture was stirred at 50°C for 10 minutes to prepare a liquid composition having a composition shown in Tables 2 to 5. Then, enzymes in the amounts shown in Tables 2 to 5 were added, and the mixture was reacted at 50°C for 2 hours at 200 rpm. Thereafter, the enzymes were deactivated by treatment at 85°C for 15 minutes. The reaction solution was emulsified at 5000 rpm for 10 minutes using a homogenizer (SILERSON, L5M-A), and then cooled to room temperature (25°C) to prepare an oil-in-water emulsified composition (pH at 25°C: 6.8).

Three 35 mL portions of the resulting emulsified composition were placed in 50 mL tubes, and samples 1, 2, and 3 for analysis were prepared as follows.

Sample 1 for analysis: Emulsified composition immediately after preparation (0 days, 25°C).

Sample 2 for analysis: Emulsified composition allowed to stand for one week in a refrigerator set at 4°C.

Sample 3 for analysis: Sample prepared by centrifuging the emulsified composition at 3000 rpm for 25 minutes (25°C). Centrifugation at 3000 rpm for 25 minutes is an acceleration condition equivalent to leaving at rest for one year (Procedia Chemistry, Volume 16, 2015, Pages 171-176).

1 mL of the samples 1 to 3 were sampled at a position 1 cm below the upper end of the emulsion phase, and diluted 100 times with purified water. For the resulting 100-times-diluted samples, the absorbance at 280 nm and the absorbance at 600 nm were measured. The fact that the protein had been solubilized was confirmed on the basis of the absorbance at 280 nm, and then the degree of emulsifiability was evaluated on the basis of the absorbance at 600 nm. The larger the absorbance at 600 nm is, the more the emulsifiability is indicated to be improved. The results are shown in Tables 2 to 5.

[Table 2]

| | | Comparative Example 1 | Example 1 | | Example 2 | | Example 3 | | Example 4 | | Example 5 | |
|---|---|---|---|---|---|---|---|---|---|---|---|---|
| Rapeseed oil (% by weight) | | 15 | 15 | | 15 | | 15 | | 15 | | 15 | |
| Pea protein material (% by weight) [Pea protein] (% by weight) | | 0.50 [0.40] | 0.50 [0.40] | | 0.50 [0.40] | | 0.50 [0.40] | | 0.50 [0.40] | | 0.50 [0.40] | |
| Sodium phosphate buffer solution (% by weight) | | Remainder | Remainder | | Remainder | | Remainder | | Remainder | | Remainder | |
| Total (% by weight) | | 100 | 100 | | 100 | | 100 | | 100 | | 100 | |
| Protein glutaminase (U/g protein) | | - | 5.0 | | 5.0 | | 5.0 | | 5.0 | | 5.0 | |
| Lipase (U/g oil or fat) | | - | 150 | | 113 | | 75 | | 38 | | 15 | |
| Absorbance 280 nm (solubility) | 0 days | 18.2 | 103.8 | 5.7x | 104.7 | 5.8x | 98.5 | 5.4x | 68.9 | 3.8x | 66.6 | 3.7x |
| | 1 week | 15.8 | 75.1 | 4.8x | 80.3 | 5.1x | 62 | 3.9x | 52.7 | 3.3x | 46.4 | 2.9x |
| | 1 year | 8.4 | 27.1 | 3.2x | 23.9 | 2.8x | 19 | 2.3x | 28.3 | 3.4x | 17.3 | 2.1x |
| Absorbance 600 nm (emulsifiability) | 0 days | 13.5 | 80.1 | 5.9x | 81.6 | 6.0x | 97.1 | 7.2x | 47.9 | 3.5x | 58.6 | 4.3x |
| | 1 week | 9.1 | 36.1 | 4.0x | 45.6 | 5.0x | 45.1 | 5.0x | 23.7 | 2.6x | 27.4 | 3.0x |
| | 1 year | 4.79 | 7.6 | 1.6x | 7.5 | 1.6x | 6.2 | 1.3x | 6.2 | 1.3x | 8.5 | 1.8x |

Representation of magnification of absorbance in Examples indicates relative value where absorbance in Comparative Example 1 is regarded as 1.

[Table 3]

| | | Comparative Example 2 | Example 6 | | Example 7 | | Example 8 | | Example 9 | |
|---|---|---|---|---|---|---|---|---|---|---|
| Rapeseed oil (% by weight) | | 15 | 15 | | 15 | | 15 | | 15 | |
| Pea protein material (% by weight) | | 0.50 | 0.50 | | 0.50 | | 0.50 | | 0.50 | |
| [Pea protein] (% by weight) | | [0.40] | [0.40] | | [0.40] | | [0.40] | | [0.40] | |
| Sodium phosphate buffer solution (% by weight) | | Remainder | Remainder | | Remainder | | Remainder | | Remainder | |
| Total (% by weight) | | 100 | 100 | | 100 | | 100 | | 100 | |
| Protein glutaminase (U/g protein) | | - | 3.8 | | 2.5 | | 1.3 | | 0.5 | |
| Lipase (U/g oil or fat) | | - | 75 | | 75 | | 75 | | 75 | |
| Absorbance 280 nm (solubility) | 0 days | 24.3 | 107.4 | 4.4x | 108 | 4.4x | 100.5 | 4.1x | 98.8 | 4.1x |
| | 1 week | 18.7 | 58.2 | 3.1x | 71.2 | 3.8x | 60 | 3.2x | 55.3 | 3.0x |
| | 1 year | 11.6 | 17 | 1.5x | 26.7 | 2.3x | 14.4 | 1.2x | 20.5 | 1.8x |
| Absorbance 600 nm (emulsifiability) | 0 days | 14.2 | 104.7 | 7.4x | 99.3 | 7.0x | 99.2 | 7.0x | 95.9 | 6.8x |
| | 1 week | 8.9 | 34.7 | 3.9x | 43.1 | 4.8x | 37 | 4.2x | 32.5 | 3.7x |
| | 1 year | 4.9 | 6 | 1.2x | 8.3 | 1.7x | 5.7 | 1.2x | 7.8 | 1.6x |

Representation of magnification of absorbance in Examples indicates relative value where absorbance in Comparative Example 2 is regarded as 1.

[Table 4]

| | | Comparative Example 3 | Example 10 | | Comparative Example 4 | Example 11 | |
|---|---|---|---|---|---|---|---|
| Rapeseed oil (% by weight) | | 15 | 15 | | 15 | 15 | |
| Pea protein material (% by weight) [Pea protein] (% by weight) | | 1.50 [1.20] | 1.50 [1.20] | | 1.00 [0.80] | 1.00 [0.80] | |
| Sodium phosphate buffer solution (% by weight) | | Remainder | Remainder | | Remainder | Remainder | |
| Total (% by weight) | | 100 | 100 | | 100 | 100 | |
| Protein glutaminase (U/g protein) | | - | 5 | | - | 5 | |
| Lipase (U/g oil or fat) | | - | 75 | | - | 75 | |
| Absorbance 280 nm (solubility) | 0 days | 56 | 201.7 | 3.6x | 28.5 | 171.9 | 6.0x |
| | 1 week | 41 | 173.4 | 4.2x | 21 | 130.3 | 6.2x |
| | 1 year | 22.7 | 68.5 | 3.0x | 13 | 44.4 | 3.4x |
| Absorbance 600 nm (emulsifiability) | 0 days | 45.8 | 149.8 | 3.3x | 18.1 | 137.2 | 7.6x |
| | 1 week | 23.9 | 93.6 | 3.9x | 7.4 | 73.4 | 9.9x |
| | 1 year | 8.16 | 14.63 | 1.8x | 3.29 | 12.36 | 3.8x |

Representations of magnification of absorbance in Examples 10 and 11 indicate relative values where absorbances in Comparative Examples 3 and 4 are regarded as 1, respectively.

[Table 5]

| | | Comparative Example 5 | Example 12 | | Comparative Example 6 | Example 13 | | Comparative Example 7 | Example 14 | |
|---|---|---|---|---|---|---|---|---|---|---|
| Rapeseed oil (% by weight) | | 5 | 5 | | 2.5 | 2.5 | | 1 | 1 | |
| Pea protein material (% by weight) [Pea protein] (% by weight) | | 0.50 [0.40] | 0.50 [0.40] | | 0.50 [0.40] | 0.50 [0.40] | | 0.50 [0.40] | 0.50 [0.40] | |
| Sodium phosphate buffer solution (% by weight) | | Remainder | Remainder | | Remainder | Remainder | | Remainder | Remainder | |
| Total (% by weight) | | 100 | 100 | | 100 | 100 | | 100 | 100 | |
| Protein glutaminase (U/g protein) | | - | 5 | | - | 5 | | - | 5 | |
| Lipase (U/g oil or fat) | | - | 75 | | - | 75 | | - | 75 | |
| Absorbance 280 nm (solubility) | 0 days | 19 | 68.9 | 3.6x | 22.5 | 59.5 | 2.6x | 19 | 49.5 | 2.6x |
| | 1 week | 12.9 | 43.8 | 3.4x | 16.8 | 48.2 | 2.9x | 13.5 | 33.3 | 2.5x |
| | 1 year | 12.6 | 17.5 | 1.4x | 8.6 | 24.7 | 2.9x | 9.6 | 17.3 | 1.8x |
| Absorbance 600 nm (emulsifiability) | 0 days | 9.9 | 57.3 | 5.8x | 15 | 34.1 | 2.3x | 11.4 | 30.3 | 2.7x |
| | 1 week | 3.2 | 19.3 | 6.0x | 8.9 | 17.1 | 1.9x | 4.9 | 15.2 | 3.1x |
| | 1 year | 5.3 | 5.8 | 1.1x | 2.71 | 5.93 | 2.2x | 3.54 | 5.27 | 1.5x |

Representations of magnification of absorbance in Examples 12, 13 and 14 indicate relative values where absorbances in Comparative Examples 5, 6 and 7 are regarded as 1, respectively.

As is apparent from Tables 2 to 5, emulsifiability was improved by treating a liquid composition containing a plant protein and an oil or fat using a protein deamidase and a lipase.

[Test Example 2]

RO water, a pea protein material, almond butter and cane sugar, and optionally sunflower oil and/or lecithin were mixed and stirred at 5000 rpm for 10 minutes using a homogenizer (SILERSON, L5M-A) to prepare liquid compositions having compositions shown in Tables 6 and 7. The amounts of total protein and total lipid in the liquid compositions are as shown in Tables 6 and 7. Enzymes in the amounts shown in Tables 6 and 7 were added to the liquid compositions, and the mixtures were stirred (200 rpm) at 50°C for 1 hour. Then, each of the mixtures was emulsified at 5000 rpm for 10 minutes, the enzymes were deactivated by heat treatment (85°C, 15 min), and the mixture was further cooled to room temperature (25°C) to prepare an oil-in-water emulsion composition (almond milk) (pH at 25°C: 6.5).

The prepared almond milk was divided into two 35-mL portions (a sample for solubility evaluation and a sample for emulsifiability evaluation) in 50-mL tubes and a 100-mL portion (a sample for foamability evaluation).

The sample for solubility evaluation and the sample for emulsifiability evaluation were prepared as samples for analysis similar to the samples 1 and 3 for analysis of Test Example 1, 100-times-diluted samples were prepared in the same manner as in Test Example 1, and the absorbance at 280 nm and the absorbance at 600 nm were measured. The results are shown in Tables 6 and 7.

In addition, the sample for foamability evaluation was foamed using a milk frother (HadinEEon, model: MMF-9401) to prepare foamed milk. The resulting foamed milk was transferred to a graduated cylinder, and the volume of the foam layer was measured to evaluate the foamability. The larger the volume is, the more the foamability is indicated to be improved. The results are shown in Tables 6 and 7.

[Table 6]

| | | Comparative Example 8 | Reference Example 1 | | Example 15 | | Example 16 | |
|---|---|---|---|---|---|---|---|---|
| Almond butter (% by weight) | | 5.8 | 5.8 | | 5.8 | | 5.8 | |
| Pea protein material (% by weight) [Pea protein] (% by weight) | | 2.8 [2.2] | 2.8 [2.2] | | 2.8 [2.2] | | 2.8 [2.2] | |
| Sunflower oil (% by weight) | | - | - | | - | | - | |
| Cane sugar (% by weight) | | 0.8 | 0.8 | | 0.8 | | 0.8 | |
| Lecithin (% by weight) | | - | 0.1 | | - | | - | |
| Water | | Remainder | Remainder | | Remainder | | Remainder | |
| Total (% by weight) | | 100 | 100 | | 100 | | 100 | |
| Total protein (% by weight) | | 3.5 | 3.5 | | 3.5 | | 3.5 | |
| Total lipid (% by weight) | | 2.8 | 2.8 | | 2.8 | | 2.8 | |
| Protein glutaminase (U/g-protein) | | - | - | | 4.2 | | 4.2 | |
| Lipase (U/g-oil or fat) | | - | - | | 75 | | 75 | |
| Protease (U/g-protein) | | - | - | | - | | 15 | |
| Absorbance 280 nm (solubility) | 0 days | 156.8 | 154.7 | 0.99x | 163.4 | 1.04x | 210.7 | 1.34x |
| | 1 year | 87.4 | 89.4 | 1.02x | 89.9 | 1.03x | 143.2 | 1.64x |
| Absorbance 600 nm (emulsifiability) | 0 days | 67.8 | 59.5 | 0.88x | 76.2 | 1.12x | 98 | 1.45x |
| | 1 year | 17.6 | 17.8 | 1.01x | 21 | 1.19x | 28.8 | 1.64x |
| Foamability (mL) | | 8 | 16 | 2x | 10 | 1.25x | 22 | 2.75x |
| Representations of magnifications of absorbance and foamability in Reference Example 1 and Examples 15 and 16 indicate relative values where absorbance and foamability in Comparative Example 8 are regarded as 1. | | | | | | | | |

[Table 7]

| | | Comparative Example 9 | Reference Example 2 | | Example 17 | |
|---|---|---|---|---|---|---|
| Almond butter (% by weight) | | 5.8 | 5.8 | | 5.8 | |
| Pea protein material (% by weight) [Pea protein] (% by weight) | | 2.8 [2.2] | 2.8 [2.2] | | 2.8 [2.2] | |
| Sunflower oil (% by weight) | | 0.4 | 0.4 | | 0.4 | |
| Cane sugar (% by weight) | | 0.8 | 0.8 | | 0.8 | |
| Lecithin (% by weight) | | - | 0.1 | | - | |
| Water | | Remainder | Remainder | | Remainder | |
| Total (% by weight) | | 100 | 100 | | 100 | |
| Total protein (% by weight) | | 3.5 | 3.5 | | 3.5 | |
| Total lipid (% by weight) | | 3.2 | 3.2 | | 3.2 | |
| Protein glutaminase (U/g-protein) | | - | - | | 4.2 | |
| Lipase (U/g-oil or fat) | | - | - | | 75 | |
| Protease (U/g-protein) | | - | - | | 15 | |
| Absorbance 280 nm (solubility) | 0 days | 158 | 165.1 | 1.04x | 198.5 | 1.26x |
| | 1 year | 79.3 | 86.2 | 1.09x | 118.4 | 1.49x |
| Absorbance 600 nm (emulsifiability) | 0 days | 69.8 | 71.4 | 1.02x | 92.9 | 1.33x |
| | 1 year | 15.5 | 16 | 1.03x | 18.4 | 1.19x |
| Foamability (mL) | | 22 | 23 | 1.05x | 40 | 1.82x |
| Representations of magnifications of absorbance and foamability in Reference Example 2 and Example 17 indicate relative values where absorbance and foamability in Comparative Example 9 are regarded as 1. | | | | | | |

As is apparent from Tables 6 and 7, emulsifiability was improved by treating a liquid composition containing a plant protein and an oil or fat using a protein deamidase and a lipase. The degree thereof was improved as compared with the case of using lecithin, which is a general-purpose emulsifier. In particular, when the protease was used in combination with the protein deamidase and the lipase, the emulsifiability was further improved.

In addition, as is apparent from Tables 6 and 7, foamability was improved as well by treating a liquid composition containing a plant protein and an oil or fat using a protein deamidase and a lipase. In particular, when a protease was used in combination with a protein deamidase and a lipase, foamability was further improved, and the degree thereof was improved as compared with the case of using lecithin, which is a general-purpose emulsifier.

**Claims**

1.  An emulsifiability improver for a liquid composition containing a plant protein and an oil or fat, comprising a protein deamidase and a lipase.

2.  The emulsifiability improver according to claim 1, wherein an amount of the lipase relative to 1 U of the protein deamidase is 1 U or more.

3.  The emulsifiability improver according to claim 1, further comprising a protease.

4.  A foamability improver for a liquid composition containing a plant protein and an oil or fat, comprising a protein deamidase and a lipase.

5.  The foamability improver according to claim 4, further comprising a protease.

6.  The foamability improver according to claim 5, wherein an amount of the protease relative to 1 U of the protein deamidase is 1 U or more.

7. A method for improving emulsifiability of a liquid composition containing a plant protein and an oil or fat, the method comprising an enzyme treatment step of allowing a protein deamidase and a lipase to act on the liquid composition.

8. The method according to claim 7, wherein the protein deamidase is used in an amount of 0.05 U or more per 1 g of the plant protein.

9. The method according to claim 7, wherein the lipase is used in an amount of 1 U or more per 1 g of the oil or fat.

10. A method for improving foamability of a liquid composition containing a plant protein and an oil or fat, the method comprising an enzyme treatment step of allowing a protein deamidase and a lipase to act on the liquid composition.

11. A method for producing an emulsion of a liquid composition containing a plant protein and an oil or fat, the method comprising an enzyme treatment step of treating the liquid composition containing the plant protein and the oil or fat with a protein deamidase and a lipase.

12. The production method according to claim 11, wherein a protease is further used in the enzyme treatment step.

13. An emulsion of a liquid composition containing a plant protein and an oil or fat, the emulsion being one obtained by the production method according to claim 11 and being a food or drink.

## INTERNATIONAL SEARCH REPORT

International application No.

**PCT/JP2023/038988**

| A. | CLASSIFICATION OF SUBJECT MATTER |
|---|---|

*C12N 9/78*(2006.01)i; *A23J 3/14*(2006.01)i; *A23L 2/00*(2006.01)i; *A23L 2/40*(2006.01)i; *A23L 2/66*(2006.01)i;
*A23L 5/00*(2016.01)i; *C07K 14/415*(2006.01)i; *C12N 9/20*(2006.01)i; *C12N 9/50*(2006.01)i; *C12P 1/00*(2006.01)i
   FI:   C12N9/78; A23J3/14; A23L2/00 J; A23L2/40; A23L2/66; A23L5/00 L; A23L5/00 M; C07K14/415; C12N9/20;
        C12N9/50; C12P1/00 A

According to International Patent Classification (IPC) or to both national classification and IPC

| B. | FIELDS SEARCHED |
|---|---|

Minimum documentation searched (classification system followed by classification symbols)

   C12N9/78; A23J3/14; A23L2/00; A23L2/40; A23L2/66; A23L5/00; C07K14/415; C12N9/20; C12N9/50; C12P1/00

Documentation searched other than minimum documentation to the extent that such documents are included in the fields searched

     Published examined utility model applications of Japan 1922-1996
     Published unexamined utility model applications of Japan 1971-2023
     Registered utility model specifications of Japan 1996-2023
     Published registered utility model applications of Japan 1994-2023

Electronic data base consulted during the international search (name of data base and, where practicable, search terms used)

     JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2022/014542 A1 (AMANO ENZYME INC.) 20 January 2022 (2022-01-20)<br>   claims 1, 8, 9, paragraphs [0023], [0025], test example 5 | 1-13 |
| X | 日世株式会社, ソフトクリームミックス [online], 28 June 2022 [retrieved on 05 December 2023], Retrieved from the Internet: <https://web.archive.org/web/20220628132222/https://www.nissei-com.co.jp/softmix/><br>   entire text, (NISSEI CO., LTD.), non-official translation (Soft cream mix [online]) | 13 |
| A | 一般社団法人 日本アイスクリーム協会, 教えて！ アイスクリーム王子 [online], 23 September 2021 [retrieved on 05 December 2023], Retrieved from the Internet: <https://web.archive.org/web/20210923115022/https://www.icecream.or.jp/iceworld/qa/10.html><br>   entire text, non-official translation (NIPPON ICE CREAM KYOKAI. Tell me! Ice cream prince [online].) | 13 |

☐ Further documents are listed in the continuation of Box C.    ☑ See patent family annex.

| | |
|---|---|
| \*   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **05 December 2023** | **19 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/038988**

| Patent document cited in search report | Publication date (day/month/year) | Patent family member(s) | Publication date (day/month/year) |
|---|---|---|---|
| WO 2022/014542 A1 | 20 January 2022 | EP 4180528 A1 claims 1. 8, 9, paragraphs [0023], [0025], test example 5 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021176284 A
- JP 2021052655 A
- WO 2014092157 A
- JP 2000050887 A
- JP 2001218590 A
- WO 2006075772 A1
- WO 2015133590 A

### Non-patent literature cited in the description

- *Procedia Chemistry*, 2015, vol. 16, 171-176